(19) Europäisches Patentamt
European Patent Office
Office européen des brevets

(11) Publication number: 0 232 822
B1

(12) EUROPEAN PATENT SPECIFICATION

(45) Date of publication of patent specification: 23.05.90

(51) Int. Cl.[5]: C 07 C 69/63, C 07 C 67/287, C 07 D 205/08

(21) Application number: 87101341.3

(22) Date of filing: 31.01.87

# (54) Process for preparing optically active fluorine-containing compounds.

(30) Priority: 03.02.86 JP 21841/86

(43) Date of publication of application: 19.08.87 Bulletin 87/34

(45) Publication of the grant of the patent: 23.05.90 Bulletin 90/21

(84) Designated Contracting States: BE CH DE ES FR GB IT LI NL SE

(56) References cited:
BULLETIN OF THE CHEMICAL SOCIETY OF JAPAN, vol. 52, no. 11, November 1979, pages 3377-3380; A. TAKAOKA et al.: "F-propene-dialkylamine reaction products as fluorinating agents"

(73) Proprietor: KANEGAFUCHI KAGAKU KOGYO KABUSHIKI KAISHA
2-4 Nakanoshima 3-chome
Kita-ku Osaka-shi Osaka-fu (JP)

(72) Inventor: Shimazaki, Masami
8-4, Nishihata 3-chome
Takasago-shi Hyogo-ken (JP)
Inventor: Ishizu, Jun-ichi
17-18, Nishihata 1-chome
Takasago-shi Hyogo-ken (JP)
Inventor: Murakami, Hiroshi
2-63, Okihama-cho Takasago-cho
Takasago-shi Hyogo-ken (JP)
Inventor: Ohashi, Takhisa
9-14, Shinoharaobanoyama-cho 3-chome Nada-ku
Kobe-shi Hyogo-ken (JP)
Inventor: Watanabe, Kiyoshi
15-41, Matsugaoka 5-chome
Akashi-shi Hyogo-ken (JP)

(74) Representative: Türk, Gille, Hrabal
Brucknerstrasse 20
D-4000 Düsseldorf 13 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 232 822 B1

Courier Press, Leamington Spa, England.

## Description

The present invention relates to a process for preparing optionally active fluorine-containing compounds.

For example, the following fluorine-containing carbapeneum antibiotic has been paid attention, and the compound has been prepared by various process steps (Japanese Tokkyo Kokai No. 84886/1984 or Japanese Tokkyo Kokai No. 216886/1984).

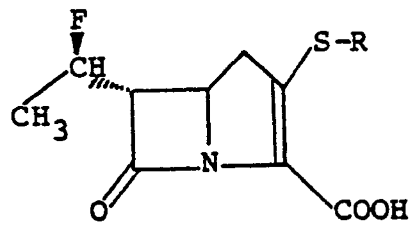

Particularly for the introduction of fluorine atom to the side chain, there is employed a reaction in which a hydroxyl group is substituted by fluorine atom with fluorinating agents. The fluorination reaction can be carried out in various reaction stages. For example, the reaction can be performed to monocyclic lactams such as azetidinone derivatives, or to dicyclic compounds prepared from the monocyclic lactams, or also to starting compounds which have no azetidinone ring.

In these known processes a hydroxyl group to be substituted by fluorine atom has common properties in structure and reactivity. Namely in case of the azetidinone derivatives the hydroxyl group is bonded to a carbon positioned at β-position of a lactam carbonyl group, and in case of the compounds having no azetidinone ring the hydroxyl group is bonded to a carbon positioned at β-position of an ester carbonyl group. Also, for obtaining the final product in which the fluorine atom has R-form, the fluorination reaction must be stereoselective. An object of the present invention is to provide a process for stereoselectively fluorinating the hydroxyl group at the β-position.

There have been known reagents which can fluorinate hydroxyl group in a single step. Examples are diethylaminosulfur trifluoride (DAST) [described in Japanese Tokkyo Kokai No. 84886/1984, or "Chemistry and Biology of β-lactam antibiotics " p366 (1984) (A. G. Brown; S. M. Roberts; The Royal Society of Chemistry, Burlington House, London WIV 0BN)]; reagents comprising hydrogen fluoride and an organic base such as pyridine; Yorovenko reagents comprising chlorotrifluoroethene and diethylamine (C. E. Arroniz et al; Prostaglandins, *16,* 47 (1978)), and the like. These reagents are highly reactive, but are remarkably instable, which requires operations at a very low temperature such as around −78°C in DAST case. In addition since the reagents are very expensive, there are large problems in industrial use.

As fluorinating agents for solving the above defects, fluorinating agents so-called Ishikawa reagents comprising hexafluoropropene and diethylalmine have been developed (Ishikawa et al; Bull. Chem. Soc. Japan, *52,* 3377 (1979)). This fluorinating agent is inexpensive and stable, and thus can be allowed to react at room temperature. This literature only disloses a fluorination reaction of D,L-mandelic acid. Further, in the reaction system SN1 reaction cannot be avoided. Therefore, the literature teaches the fact that when fluorinating hydroxyl group bonded to an asymmetric carbon atom by using the Ishikawa reagent, occurrence of racemization cannot be avoided.

From the present inventor's experimental study, almost all of the reaction products prepared by fluorinating ethyl L-mandelate with the Ishikawa reagent were racemized (referring to Reference Example 7, wherein only 12% e.e. of optical activity being kept). Considering the fact that the position of hydroxyl group of ethyl L-mandelate is at α-position of ester carbonyl group, and the fact that elimination reaction mechanisms between the α-hydroxyl group and the β-hydroxyl group are different, the present inventors have studied fluorination of hydroxyl group at the β-position of an ester carbonyl group or a lactam carbonyl group of an azetidinone derivative, and then it has been found that the hydroxyl group can be stereoselectively fluorinated according to inverse substitution reaction when the reaction temperature is controlled at a temperature of not more than room temperature.

According to the present invention, there is provided a process for preparing an optically active fluorine-containing compound, which comprises reacting a stereoisomer of a compound which has an ester carbonyl group or a lactam carbonyl group and has a hydroxyl group bonded to an asymmetric carbon atom at β-position of the carbonyl group (hereinafter referred to as "β-hydroxyl compound"), with a fluorinating agent which is a reaction product of hexafluoropropene and diethylamine in an amount of 1 to 5 moles to 1 mole of the stereoisomer by controlling a temperature of the reaction system within a range from −50° to 25°C to substitute the hydroxyl group by fluorine atom; said optically active fluorine-containing compound has a configuration inverse to that of the starting stereoisomer.

The starting compound, i.e. optically active β-hydroxyl compound of the present invention is not

particularly limited insofar as to satisfy the above definition. Typical examples of the β-hydroxyl compound are, for instance, an optically active ester of 3(S) or 3(R)-hydroxybutyric acid of the formula (I):

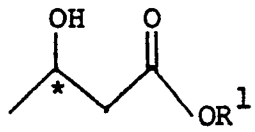

(I)

wherein $R^1$ is an alkyl group of $C_1$ to $C_4$, or an optically active 3-[1(S) or 1(R)-hydroxyethyl]azetidine-2-one derivative of the formula (II):

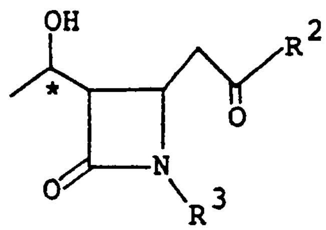

(II)

wherein $R^2$ is an alkoxy group of $C_1$ to $C_4$, an arylalkyloxy group of $C_7$ to $C_8$, a group of the formula (III):

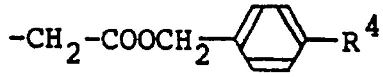

(III)

in which $R^4$ is hydrogen atom or nitro, or a group of the formula (IV):

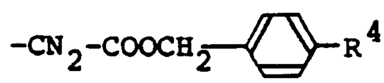

(IV)

in which $R_4$ is as defined above; $R_3$ is hydrogen atom or a protective group of the amino group. Examples of the substituent $R^2$ are, for instance, alkoxy groups such as methoxy, arylalkyloxy groups such as benzyloxy, p-nitrobenzyloxy and p-anisyloxy. The preferable group of the formula (III) or (IV) is a group in which $R_4$ is nitro.

According to the present invention, from the optically active ester of 3-hydroxybutyric acid (I) an optically active ester of 3-fluorobutyric acid of the formula (V):

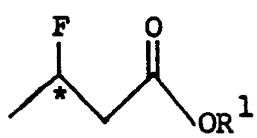

(V)

wherein $R^1$ is as defined above and its configuration is inverted to that of the starting ester of 3-hydroxybutyric acid (I) can be obtained. Also from the optically active 3-[1-hydroxyethyl]azetidinone derivative (II) an optically active 3-[1-fluoroethyle]azetidine-2-one derivative of the formula (VI):

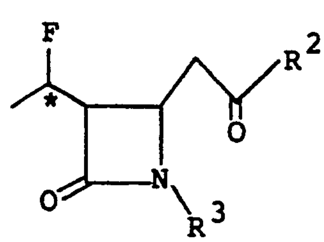

(VI)

wherein $R^2$ and $R^3$ are as defined above and its configuration is inverted to that of the starting optically active derivative (II).

For example, a fluorine-containing compound (Va):

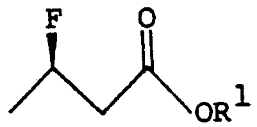

(Va)

can be obtained from a β-hydroxyl compound (Ia):

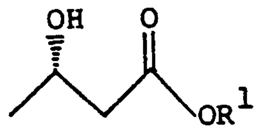

(Ia)

Also, a fluorine-containing compound (VIa):

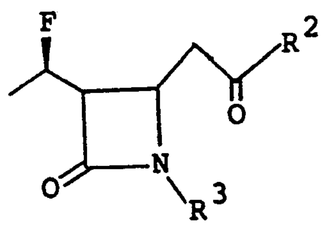

(VIa)

can be prepared from a β-hydroxyl compound (IIa):

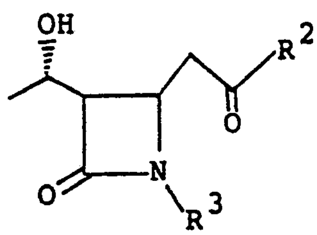

(IIa)

The azetidinone derivative can be prepared by, for example, acting acetic acid anhydride on 3-[1(R)-o-dimethyl-t-butylsilyloxyethyl]-4-trimethylsilyloxy-azetidine-2-one in an organic solvent in the presence of a base to prepare 3-[1(R)-o-dimethyl-t-butylsilyloxyethyl)]-4-actoxyazetidine-2-one, and then to 4-position of the product a desired group is introduced according to methods described in the Reference Examples, or other methods.

The above β-hydroxyl compounds (I), (II) are compounds which are fluorinated in the course of preparation of the fluorine-containing carbapeneum. Accordingly, the β-hydroxyl compounds are preferably S-form.

Particularly, the 3-[1-hydroxyethyl]azetidine-2-one derivative contains a highly reactive diazo group, and thus according to the conventional processes the derivative is avoided to use as a compound to be fluorinated. However according to the present invention the derivative (II) can also be fluorinated to produce a desired optically active fluorine-containing compound. Therefore, when employing the present invention, the process steps of the above conventional processes of Japanese Tokkyo Kokai No. 84886/1984 and No. 216886/1984 can be shortened by two steps.

The reaction temperature of the fluorination should be kept within a range from −50° to 25°C during all over the reaction. Considering the facts that the fluorine substitution reaction is proceeded at around −20°C and that the reaction is an (strong) exothermic reaction, it is preferable to start the reaction at −50°C. In this case, the reaction temperature can be kept below 25°C during all over the fluorination reaction and be

sufficiently proceeded, even if any temperature regulation is absent. Of course the reaction temperature may be regulated at around −20°C. Though in case of DAST a fluorination reaction must be carried out at a very low temperature of −78°C, the fluorination reaction is practically started from a temperature −50° to −40°C or higher in case of the Ishikawa reagent. In addition, since racemization tends to occur at a relatively high temperature, an optical purity of the product is improved to some extent when the reaction temperature is kept at not more than 0°C.

Solvents used in the fluorination reaction are solvents which do not affect the reaction, and are, for example, methylene chloride, ethyl ether, or the like.

The fluorinating agent used in the present invention is a reaction product of hexafluoropropene and diethylamine. The Ishikawa reagents are incorporated into the present invention. The amount of the fluorinating agent is 1 to 5 moles, preferably 1 to 2 moles, to 1 mole of the stereoisomer of the β-hydroxyl compound.

The present invention is more specifically described and explained by means of the following Reference Examples and Examples, and it is to be understood that the present invention is not limited to the Examples, and various changes and modifications may be made in the invention without departing from the spirit and scope thereof.

Example 1

(Synthesis of methyl 3(S)-fluorobutyrate)

25 g (0.21 mole) of methyl 3(R)-hydroxybutyrate having $[\alpha]_D^{20}$ (c = 1.0 $CHCl_3$) of −48° dissolved in 100 ml of methylene chloride was sufficiently cooled in an ice bath, and thereto 73 ml (0.21 mole) of diethylaminehexafluoropropene reagent was added dropwise for 70 minutes with stirring so as to keep the temperature of the mixture below 10°C. The stirring was continued for 3 hours at around 5°C. After confirming disappearance of the starting material by TLC (silica gel; toluene/acetone = 9/1), 20 g of sodium bicarbonate was added and the mixture was sufficiently stirred, and then 100 ml of ice water was added. After the methylene chloride layer was collected, and the layer was washed with water an an aqueous solution of NaCl, and then dried on anhydrous magnesium sulfate, followed by distillation with 30 cm Widmer distillation column. In the distillation a very small amount of methyl crotomate was obtained as a fraction of b.p. 26° to 33°C/30 mmHg and 14 g of the desired product, i.e. methyl 3(S)-fluorobutyrate was obtained as a fraction of b.p. 40° to 42°C/30 mmHg.

$[\alpha]_D^{20}$ + 6.5° (c = 2, $CHCl_3$)

$[\alpha]_D^{25}$ + 10.5° (c = 2, MeOH)

$^1$H—NMR ($CDCl_3$, 90 MHz)

δ 1.41 (3H, d-d, $J_{3,4}$ = 6.5 Hz, $J_{F,4}$ = 23.4 Hz, $CH_3$) 2.1—2.9 (2H, m, $CH_2$) 3.64 (3H, s, $OCH_3$) 4.98 (1H, d-q, $J_{3,4}$ = 6.5 Hz, $J_{F,3}$ = 47.4 Hz, $CH$)

Example 2

(Synthesis of methyl 3(R)-fluorobutyrate)

The same procedures as in Example 1 were repeated except that 25 g (0.21 mole) of methyl 3(S)-hydroxybutyrate having $[\alpha]_D^{20}$ (c = 1.0 $CHCl_3$) of +41.0° was employed instead of methyl 3(R)-hydroxybutyrate and that the fluorinating agent (0.252 mole) was added for 25 minutes while keeping a temperature of the reaction mixture within a range of −6° to 6°. The obtained crude product was distilled under a reduced pressure which was a little higher than that of Example 1 to give 12 g of methyl 3(R)-fluorobutyrate as a fraction of bp 56° to 57°C/60 mmHg.

$[\alpha]_D^{25}$ − 7.8° (c = 2, MeOH)

$^1$H—NMR ($CDCl_3$, 90 MHz): the same signals as those of the product in Example 1.

Example 3

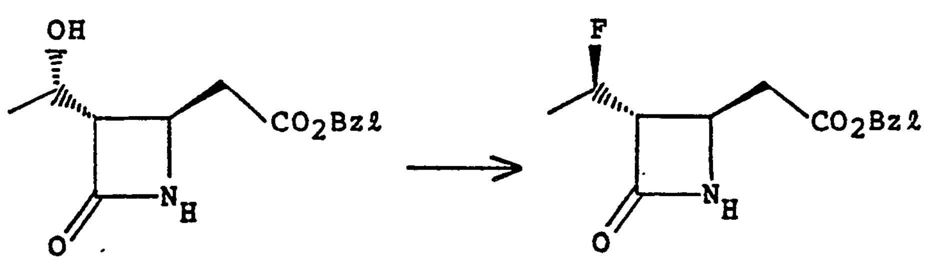

1.5 g (5.70 mmole) of (3S,4R)-4-benzyloxy-carbonylmethyl-3-[1(S)-hydroxyethyl]azetidine-2-one was dissolved in 20 ml of dry methylene chloride, and cooled to −60° to −70°C in dry ice-acetone bath. To the cooled mixture 1.51 ml (7.98 mmole) of hexafluoropropenediethylamine reagent was added dropwise, and then stirred over a night while naturally raising the temperature of the mixture to room temperature (final temperature: 10°C). The resulting mixture was cooled to −20°C with stirring, poured to 100 ml of saturated

sodium hydrogen carbonate, and then stirred for 30 minutes at room temperature. After extracting with 50 ml of ethyl acetate three times, the ethyl acetate layer was washed with water, dried on anhydrous magnesium sulfate, and condensed to give 2.9 g of a yellow oily crude fluorinated product. The crude product was subjected to silica gel column chromatography by using hexane-acetone (4:1) to give 630 mg of (3R,4R)-4-benzyloxycarbonylmethyl-3-[1(R)-fluoroethyl]azetidine-2-one in a yellow oily form (yield: 42% by mole).

Example 4

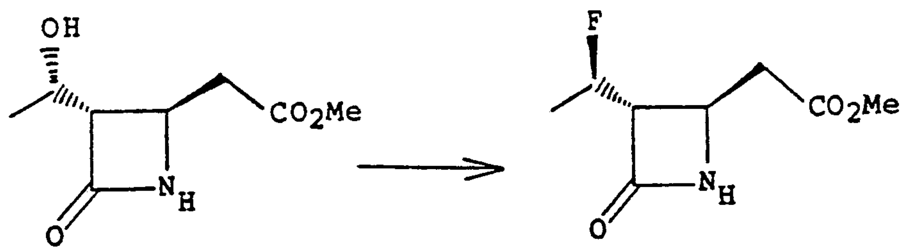

The same procedures as in Example 3 were repeated except that (3S,4R)-4-methoxycarbonylmethyl-3-[1(S)-hydroxyethyl]azetidine-2-one was employed instead of (3S,4R)-4-benzyloxycarbonylmethyl-3-[1(S)-hydroxyethyl]azetidine-2-one to give (3R,4R)-4-methoxycarbonylmethyl-3-[1(R)-fluoroethyl]azetidine-2-one (yield: 40% by mole).

Example 5

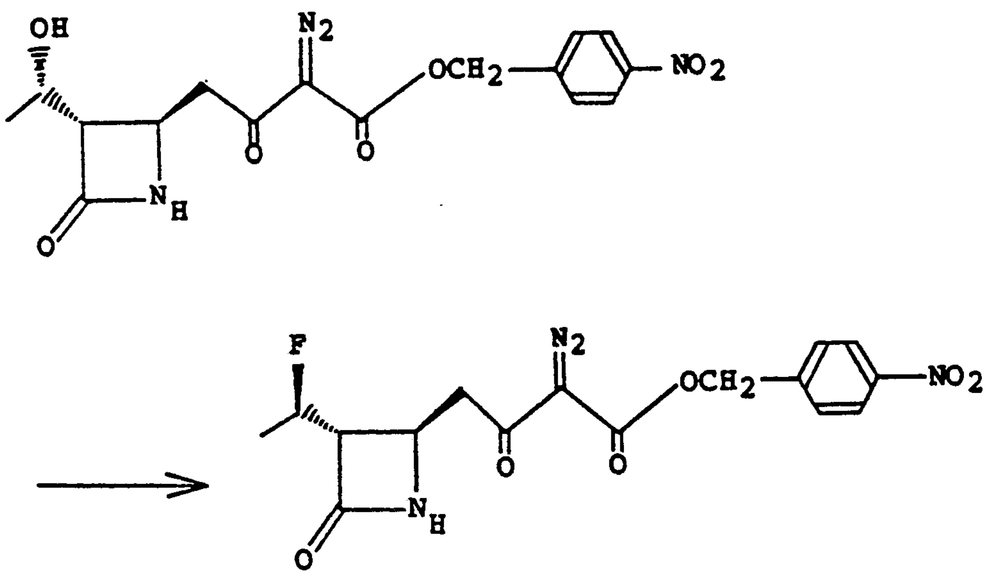

323 mg (0.86 mmole) of (3S,4R)-4-[3-(4-nitrobenzyloxycarbonyl)-3-diazo-2-oxopropane-1-yl]-3-[1(S)-hydroxyethyl]azetidine-2-one was dissolved in 5 ml of dry methylene chloride, and cooled to −20°C in dry ice-acetone bath. To the cooled mixture 228 μl (1.20 mmole) of hexafluoropropene-diethylamine reagent was added dropwise, and then stirred for a night while naturally raising the temperature of the mixture to room temperature. The reaction mixture was poured to 20 ml of a cooled aqueous solution of saturated sodium hydrogen carbonate, and stirred for 30 minutes. After extracting with 25 ml of ethyl acetate three times, washing with water, and dried on anhydrous magnesium sulfate, the solution was condensed.

The resulting yellow oily residue was subjected to silica gel column chromatography by using hexane-acetone (1:0 to 2:1) to obtain 120 mg of (3R,4R)-4-[3-(4-nitrobenzyloxycarbonyl)-3-diazo-2-oxopropane-1-yl]-3-[1(R)-fluoroethyl]azetidine-2-one in a yellow oily form (yield: 37% by mole).

$^1$H—NMR ($CDCl_3$, 90 MHz)

δ 1.45 (dd, 3H), 3.04 (ddd, 1H), 3.06 (dd, 1H), 3.44, (dd, 1H), 4.04 (ddd, 1H), 4.98 (dq, 1H), 5.38 (s, 2H), 6.17 (br s, 1H), 7.58 (d, 2H), 8.31 (d, 2H)

IR (neat): 2135 $cm^{-1}$

Reference Example 1

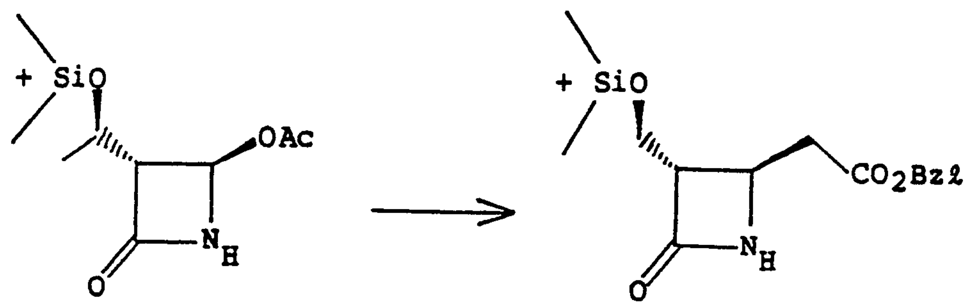

5 g (17.4 mmole) of (3R,4R)-4-acetoxy-3-[1(R)-dimethyl-t-butylsiloxyethyl]azetidine-2-one was dissolved in 150 ml of dry tetrahydrofuran, and thereto 3.09 ml (24.4 mmole) of trimethylchlorosilane and 4.12 ml (29.6 mmole) of triethylamine were added at room temperature, and then stirred for 4 hours. After filtering off the resulting salt, an excess amount of triethylamine was removed under reduced pressure, and thereto 100 ml of dry tetrahydrofuran was again added. After filtering off the insoluble residue, the solution was evaporated under reduced pressure to dryness to give 6.37 g of N-trimethylsilyl derivative in a yellow oily form.

Separately, 4.7 g (31.3 mmole) of benzyl acetate was dissolved in 45 ml of dry methylene chloride, and thereto 4.8 ml (34.4 mmole) of triethylamine and 6.66 ml (34.4 mmole) of trimethylsilyl trifluoromethylsulfonate were added, and then stirred for 30 minutes in ice bath under nitrogen atmosphere. To the transparent solution, 250 ml of dry hexane was added and stirred for 10 minutes. After removing the precipitated oily residue by decantation, the hexane solution was evaporated to dryness to give an oily residue. The residue was dissolved in 200 ml of dry hexane, and the insoluble residue was again removed by decantation. The obtained hexane solution was condensed under reduced pressure to give 6.93 g of silyl enolate in a colorless oily form.

The N-trimethylsilyl derivative was dissolved in 100 ml of dry methylene chloride, and thereto silyl enolate dissolved in 15 ml of dry methylene chloride was added at room temperature under nitrogen atmosphere. To the transparent yellow solution 0.5 ml of trimethylsilyl trifluoromethylsulfonate was added, and stirred for a night. After washing the organic layer with a saturated sodium hydrogen carbonate solution and then a saturated NaCl solution, the solution was dried on anhydrous magnesium sulfate, and then condensed under reduced pressure to give 9.24 g of a yellow oily product. The crude product was subjected to silica gel column chromatography by using hexane-acetone (10%—20%) to give 4.6 g of (3S,4R)-4-benzyloxycarbonylmethyl-3-[1(R)-dimethyl-t-butylsilyloxyethyl]azetidine-2-one in a pale yellow solid form (yield: 70% by mole).

Reference Example 2

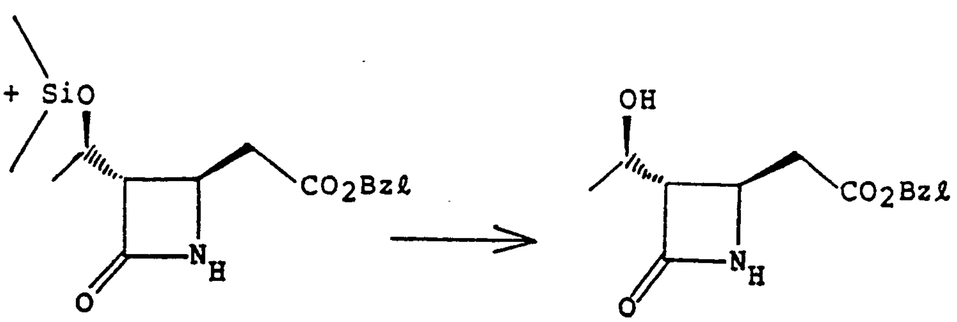

4.68 g of (3S,4R)-4-benzyloxycarbonylmethyl-3-[1(R)-dimethyl-t-butylsilyloxyethyl]azetidine-2-one was dissolved in 130 ml of tetrahydrofuran, and thereeto 39 ml of 1N—HCl was added, and then stirred for a night at room temperature. To the mixture which was cooled in ice bath 3.26 g of sodium hydrogen carbonate dissolved in a small amount of water was added with stirring, and stirred for 30 minutes at room temperature. After extracting with 50 ml of ethyl acetate three times, washing with water and drying on anhydrous magnesium sulfate, the solution was condensed under reduced pressure to give 4.28 g of a pale yellow oily residue. The residue was subjected to silica gel column chromatography by using hexane-acetone (1:1) to give 2.98 g of (3S,4R)-4-benzyloxycarbonylmethyl-3-[1(R)-hydroxyethyl]azetidine-2-one in a pale yellow oily form (yield: 91% by mole).

Reference Example 3

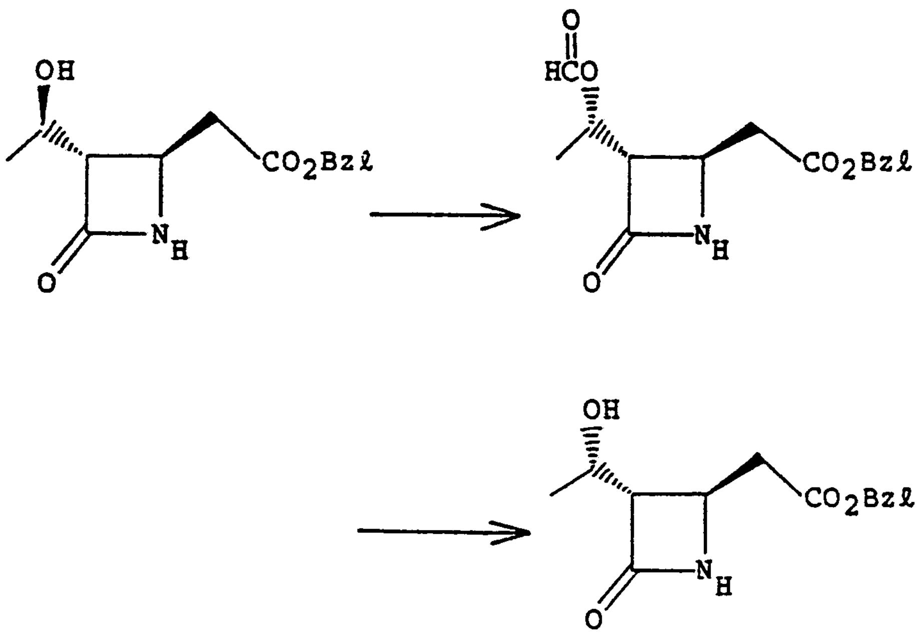

2.27 g (8.62 mmole) of (3S,4R)-4-benzylcarbonylmethyl-3-[1(R)-hydroxyethyl]azetidine-2-one was dissolved in 30 ml of dry tetrahydrofuran, and thereto 2.26 g (8.62 mmole) of triphenylphosphine was added under nitrogen atmosphere. To the mixture cooled in ice bath 0.98 ml (25.8 mmole) of formic acid was added, and then 1.36 ml (8.62 mmole) of diethyl azodicarboxylate was added dropwise. After stirring for 2 hours at room temperature, 2.26 g of triphenylphosphine and 1.36 ml of diethyl azodicarboxylate were again added, and stirred for a night. After filtering off the insoluble residue by using Hyflo Super-Cel as an assistant, the solution was condensed under reduced pressure to obtain a yellow oily residue. To the residue 20 ml of ether was added, and the resulting precipitate was filtered off, and then the solution was evaporated to dryness to give 10.25 g of a brown oily product. The crude formate was dissolved in 150 ml of methanol, and thereto 80 ml of 1N—HCl was added, and then stirred for 4 hours at room temperature. After adding 100 ml of a phosphate buffer solution, methanol was removed under reduced pressure. The aqueous layer was extrated with 100 ml of ethyl acetate three times. After washing the organic layer with water, the solution was dried on anhydrous sodium sulfate and condensed to give 9.65 g of a brown oily product. The crude product was subjected to silica gel column chromatography by using hexane-acetone (2:1) to give 1.6 g of (3S,4R)-4-benzyloxycarbonylmethyl-3-[1(S)-hydroxyethyl]azetidine-2-one in a yellow oily form (yield: 70% by mole).

Reference Example 4

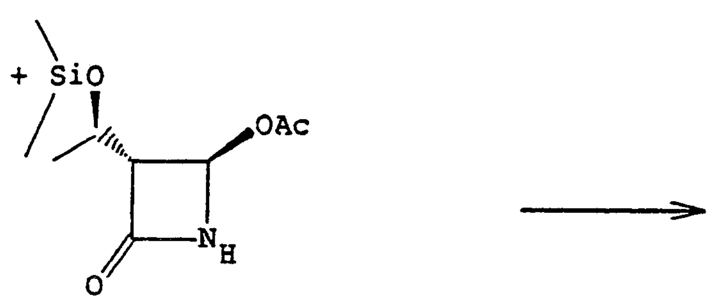

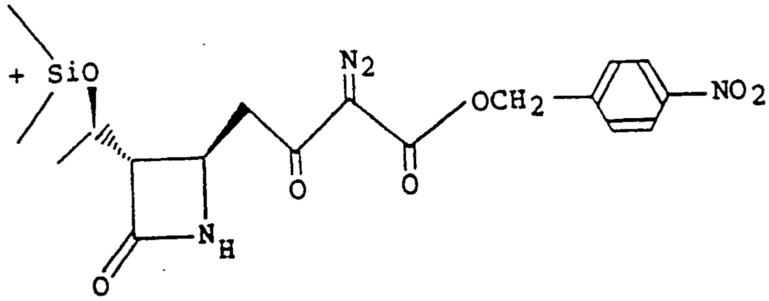

6.87 g of p-nitrobenzyl α-diazoacetoacetate was suspended in 10 ml of dry methylene chloride, and thereto 9.93 ml of triethylamine was added. To the mixture which was cooled in ice bath 5.55 ml of trimethylsilyl trifluoromethylsulfonate was added dropwise under nitrogen atmosphere, and stirred for 30 minutes at the temperature. 200 ml of dry hexane was added to the transparent yellow solution and stirred for 10 minutes. After removing the precipitated oily residue by decantation, the hexane solution was evaporated to dryness to give a yellow solid. The solid was dissolved in 200 ml of dry hexane, and the insoluble residue was again removed by decantation. The obtained hexane solution was evaporated under reduced pressure to dryness to give 8.1 g of silyl enolate in a yellow solid form.

After drying a mixture of 5 g of (3R,4R)-4-acetoxy-3-[1(R)-dimethyl-t-butylsilyloxyethyl]azetidine-2-one and 0.4 g of molten zinc chloride in vacuum, the dried mixture was dissolved in 100 ml of dry methylene chloride. To the solution silyl enolate dissolved in 50 ml of dry methylene chloride was added under nitrogen atmosphere at room temperature, and stirred for a night. The organic layer was condensed under reduced pressure to give about 10 g of a yellow oily product. The crude product was subjected to silica gel column chromatography by using hexane-acetone (10%—50%) to give 5.05 g of (3R,4R)-4-[3-(4-nitrobenzyloxycarbonyl)-3-diazo-2-oxopropane-1-yl]-3-[1(R)-dimethyl-t-butylsilyloxyethyl]-azetidine-2-one in a yellow foam (yield: 59% by mole).

Reference Example 5

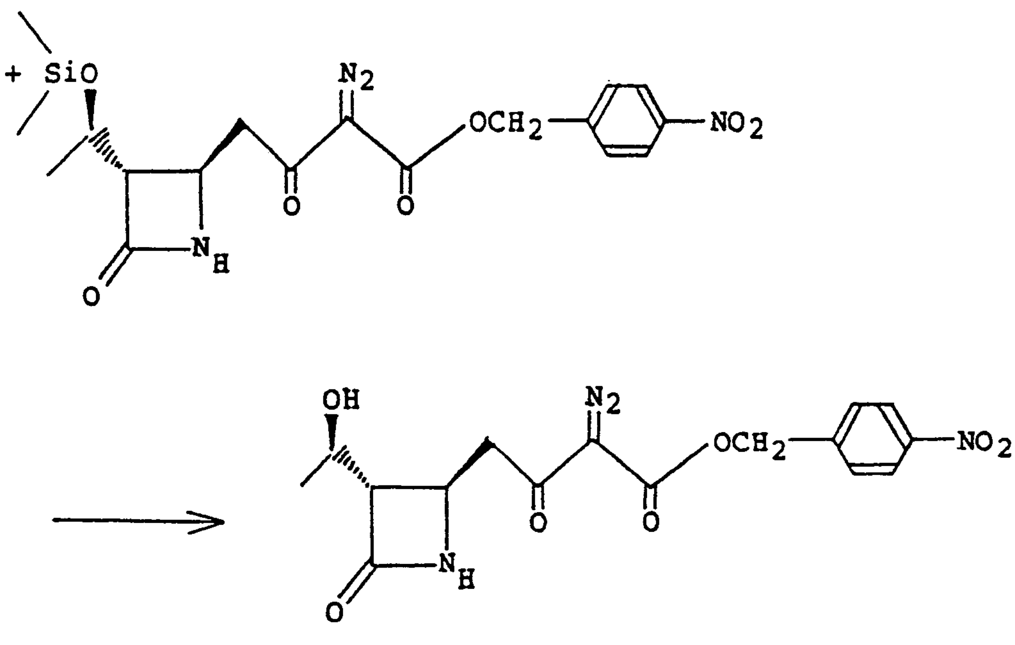

5.05 g of (3S,4R)-4-[3-(4-nitrobenzyloxycarbonyl)-3-diazo-2-oxopropane-1-yl]-3-1(R)-dimethyl-t-butylsilyloxyethyl]azetidine-2-one was dissolved in 70 ml of methanol, and thereto 14 ml of 1N—HCl was added, and then stirred for 3 hours at room temperature. The precipitated solid was collected by filtration, washed with a small amount of cold methanol-water (9:1), and dried to give 2.12 g of (3S,4R)-4-[3-(4-nitrobenzyloxycarbonyl)-3-diazo-2-oxopropane-1-yl]-3-[1(R)-hydroxyethyl]azetidine-2-one in a yellow solid form (yield: 54.7% by mole).

Reference Example 6

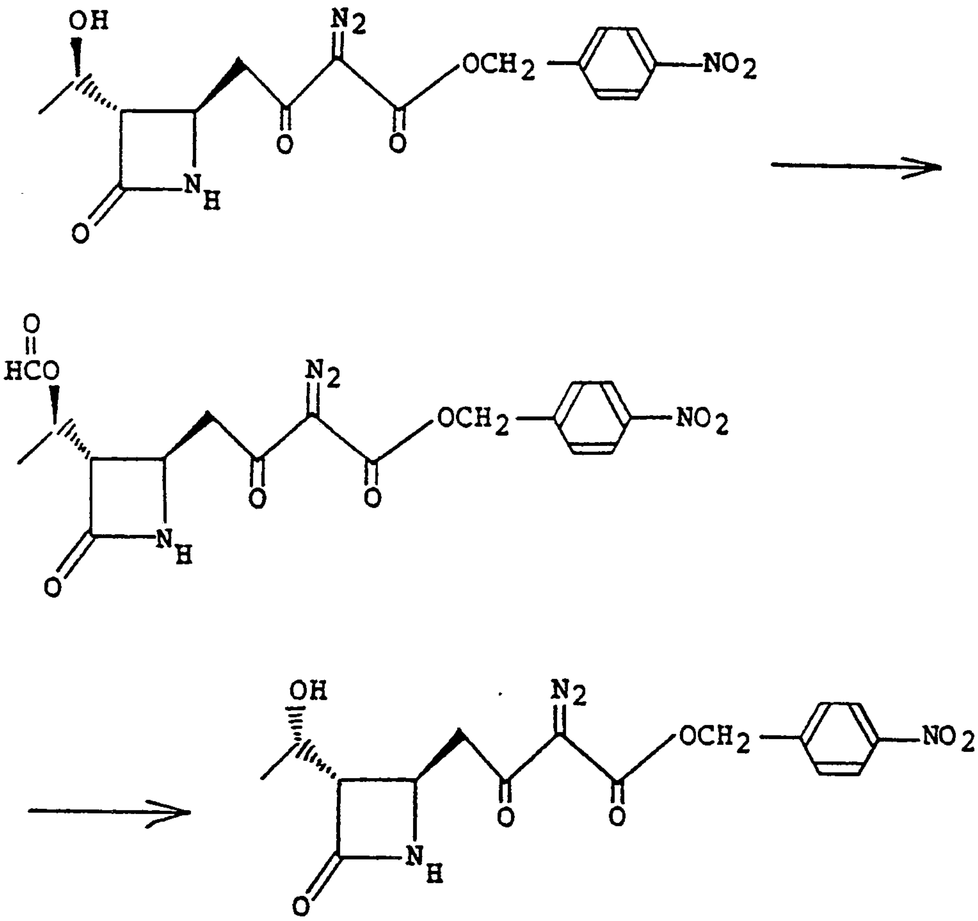

2.05 g of (3S,4R)-4-[3-(4-nitrobenzyloxycarbonyl)-3-diazo-2-oxopropane-1-yl]-3-[1(R)-hydroxyethyl]azetidine-2-one was dissolved in 25 ml of dry tetrahydrofuran, and thereto 2.50 g of triphenylphosphine was added under nitrogen atmosphere. To the mixture which was cooled in ice bath, 0.54 ml of formic acid was added, and 1.5 ml of diethyl azodicarboxylate was added dropwise, and then stirred for a night at room temperature. After filtering off the insoluble residue by using Hyflo Super-Cel as an assistant, the solution was condensed under reduced pressure to obtain a yellow oily residue. To the residue 50 ml of hexane-ethyl acetate (1:1) was added, and the resulting precipitate was filtered off, and then the solution was evaporated to dryness to give a yellow oily product. The crude formate was dissolved in 25 ml of methanol, and thereto 10 ml of 1N—HCl was added, and then stirred for 3 hours at room temperature. After adding 50 ml of a phosphate buffer solution (pH 7), methanol was removed under reduced pressure. The aqueous layer was extracted with 25 ml of ethyl acetate three times. After washing the organic layer with water, the solution was dried on anhydrous magnesium sulfate and evaporated under reduced pressure to dryness to give 9.5 g of a yellow oily residue. The residue was subjected to silica gel column chromatography by using hexane-acetone (2:1) to give 1.11 g of (3S,4R)-4-[3-(4-nitrobenzyloxycarbonyl)-3-diazo-2-oxopropane-1-yl]-3-[1(S)-hydroxyethyl]azetidine-2-one in a yellow oily form (yield: 54.1% by mole).

Reference Example 7

200 ml of a pure ethyl L-mandelate was dissolved in 20 ml of methylene chloride, and thereto 2 equivalents of the Ishikawa reagent was added, and then the fluorination reaction was carried out for 24 hours at a temperature of not more than 25°C. To the reaction mixture 50 ml of a 0.5 M phosphate buffer solution (pH 6.8) was added. After extracting with 100 ml of methylene chloride, the organic layer was dried on anhydrous magnesium sulfate, and then the solvent was distilled off. The residue was dissolved in 50 ml of dioxane, and thereto 5 equivalents of a 10% $H_2SO_4$ was added. The resulting solution was stirred for 4 hours at 80°C to hydrolyze the ester to a carboxylic acid completely. As described in Examples 3 and 4, the optical activity can be kept in these conditions.

After isolating the carboxylic acid, the carboxylic acid was converted to the corresponding acid chloride by using thionyl chloride, and the acid chloride was reacted with (S)-α-methylbenzylamine to give

a diastereomer of the corresponding amido.

As a result of gas chromatography by using a capillary column, the obtained fluorinated compound was a mixture of isomers (56:44; i.e. 12% e.e.).

## Claims

1. A process for preparing an optically active fluorine-containing compound, which comprises reacting a stereoisomer of a compound which has an ester carbonyl group or a lactam carbonyl group and has a hydroxyl group bonded to an asymmetric carbon atom at β-position of the carbonyl group, with a fluorinating agent which is a reaction product of hexafluoropropene and diethylamine in an amount of 1 to 5 moles to 1 mole of the stereoisomer by controling a temperature of the reaction system within a range from −50° to 25°C to substitute the hydroxyl group by fluorine atom; said optically active fluorine-containing compound has a configuration inverse to that of the starting stereoisomer.

2. The process of Claim 1, wherein the compound having the hydroxyl group is an ester of 3-hydroxybutyric acid of the formula (I):

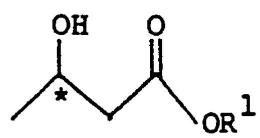

(I)

wherein $R^1$ is an alkyl group of $C_1$ to $C_4$, or a 3-[1-hydroxyethyl]azetidine-2-one derivative of the formula (II):

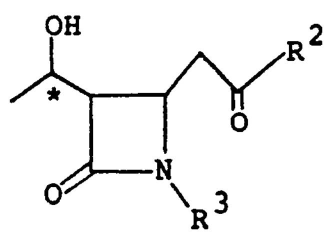

(II)

wherein $R^2$ is an alkoxy group of $C_1$ to $C_4$, an arylalkyloxy group of $C_7$ to $C_8$, a group of the formula (III):

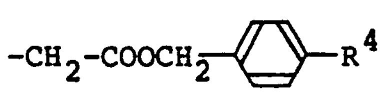

(III)

in which $R^4$ is hydrogen atom or nitro, or a group of the formula (IV):

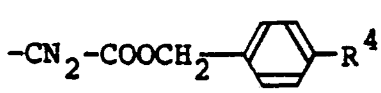

(IV)

in which $R^4$ is as defined above; $R^3$ is hydrogen atom or a protective group of the amino group, and the obtained fluorine-containing compound is an ester of 3-fluorobutyric acid of the formula (V):

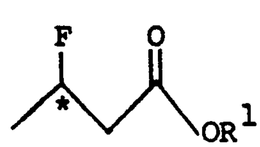

(V)

wherein $R^1$ is as defined above and the configuration of the fluorinated carbon is inverted, or a 3-[1-fluoroethyle]azetidine-2-one derivative of the formula (VI):

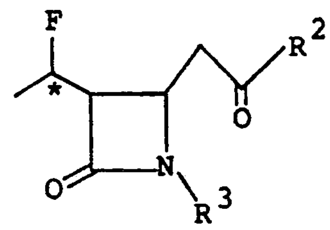

(VI)

wherein $R^2$ and $R^3$ are as defined above and the configuration of the fluorinated carbon is inverted.

3. The process of Claim 2, wherein the compound having the hydroxyl group is an ester of 3(S)-hydroxylbutyric acid of the formula (Ia):

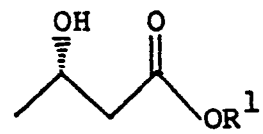

(Ia)

wherein $R^{1'}$ is as defined above, and the obtained fluorine-containing compound is an ester of 3(R)-fluorobutyric acid of the formula (Va):

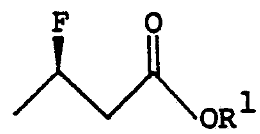

(Va)

wherein $R^1$ is as defined above.

4. The process of Claim 2, wherein the compound having the hydroxyl group is a 3-[1(S)-hydroxyethyl]-azetidine-2-one derivative of the formula (IIa):

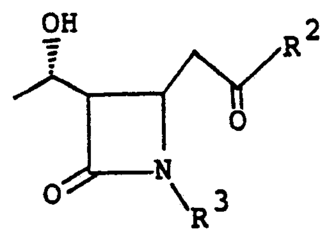

(IIa)

wherein $R^2$ and $R^3$ are as defined above, and the obtained fluorine-containing compound is a 3-[1(R)-fluoroethyl]-azetidine-2-one derivative of the formula (VIa):

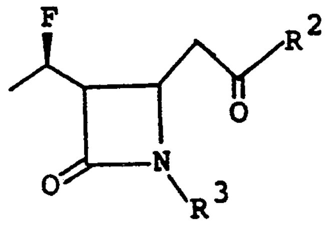

(VIa)

wherein $R^2$ and $R^3$ are as defined above.

**Patentansprüche**

1. Verfahren zur Herstellung einer optisch aktiven Fluor-enthaltenden Verbindung, welches umfaßt Umsetzen eines Stereoisomeren einer Verbindung, welche eine Ester-Carbonylgruppe oder eine Lactam-Carbonylgruppe hat und eine Hydroxylgruppe gebunden an ein asymmetrisches Kohlenstoffatom in der β-Position zur Carbonylgruppe hat, mit einem Fluorierungsmittel, welches das Reaktionsprodukt von Hexafluorpropen und Diethylamin ist, in einer Menge von 1 bis 5 Molen au 1 Mol des Stereoisomeren durch Kontrollieren der Temperatur des Reaktionssystems innerhalb eines Bereiches von −50° bis 25°C, um die Hydroxylgruppe durch Fluoratom zu substituieren; die optisch aktive Fluor-enthaltende Verbindung hat eine Konfiguration invers zu der des Ausgangs-Stereoisomeren.

2. Verfahren nach Anspruch 1, worin die Verbindung mit der Hydroxylgruppe ein Ester ist der 3-Hydroxybutansäure der Formel (I):

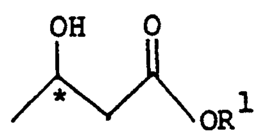

(I)

worin $R^1$ eine Alkylgruppe von $C_1$ bis $C_4$ oder ein 3-[1-Hydroxyethyl]-azetidin-2-on-Derivat der Formel (II) ist:

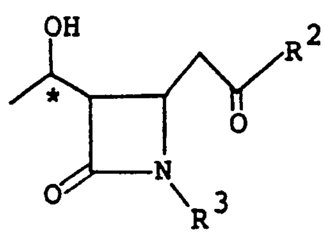

(II)

worin $R^2$ ist eine Alkylgruppe von $C_1$ bis $C_4$, eine Arylalkyloxygruppe von $C_7$ bis $C_8$, eine Gruppe der Formel (III):

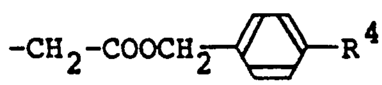

(III)

worin $R_4$ ist wasserstoffatom oder Nitro, oder eine Gruppe der Formel (IV):

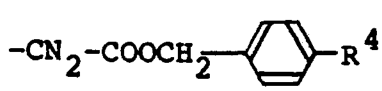

(IV)

worin $R^4$ wie oben definiert ist; $R^3$ Wasserstoffatom ist oder eine Schutzgruppe der Aminogruppe, und die erhaltene Fluor-enthaltende Verbindung ein Ester der 3-Fluorbutansäure der Formel (V) ist:

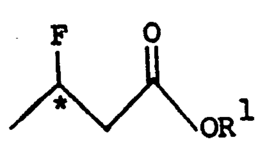

(V)

worin $R^1$ wie oben definiert ist und die Konfiguration des fluorierten Kohlenstoffs invertiert ist, oder ein 3-[1-Fluorethyl]-azetidin-2-on-Derivat der Formel (VI):

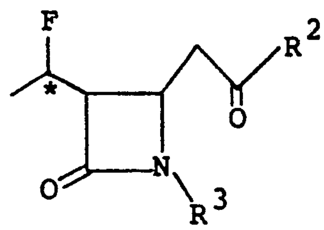

(VI)

worin $R^2$ und $R^3$ wie oben definiert sind und die Konfiguration des fluorierten Kohlenstoffs invertiert ist.

3. Verfahren nach Anspruch 2, worin die Verbindung mit der Hydroxylgruppe ein Ester der 3(S)-Hydroxylbutansäure der Formel (Ia) ist:

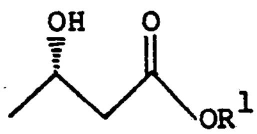

(Ia)

worin $R^1$ wie oben definiert ist und die erhaltene Fluor-enthaltende Verbindung ein Ester der 3(R)-Fluorbutansäure der Formel (Va) ist:

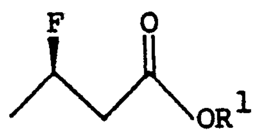

(Va)

worin $R^1$ wie oben definiert ist.

4. Verfahren nach Anspruch 2, worin die Verbindung mit der Hydroxylgruppe ein 3-[1(S)-Hydroxyl]-azetidin-2-on-Derivat der Formel (IIa) ist:

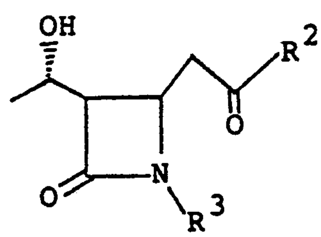

(IIa)

worin $R^2$ und $R^3$ wie oben definiert sind und die erhaltene fluor-enthaltende Verbindung ein 3-[1(R)-Fluorethyl]-azetidin-2-on-Derivat der Formel (VIa) ist:

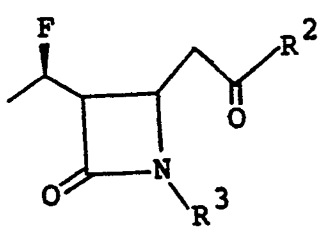

(VIa)

worin $R^2$ und $R^3$ wie oben definiert sind.

**Revendications**

1. Procédé de préparation d'un composé fluoré optiquement actif, dans lequel on fait réagir un stéréoisomère d'un composé comportant un groupe carbonyle d'ester ou un groupe carbonyle d'ester ou un groupe carbonyle de lactame, et un groupe hydroxyle lié à un atome de carbone asymétrique en position β par rapport au groupe carbonyle, avec un agent de fluoration qui est un produit de réaction de l'hexafluoropropène et de la diéthylamine, selon une quantité de 1 à 5 moles, pour 1 mole du stéréoisomère, en ajustant la température du système réactionnel, à 50° jusqu'à 25°C, afin de substituer le groupe hydroxyle par un atome de fluor; ce composé fluoré optiquement actif, ayant une configuration inverse de celle de stéréoisomère de départ.

2. Procédé selon la revendication 1, dans lequel le composé comportant le groupe hydroxyle, est un ester de l'acide 3-hydroxybutyrique, de formule (I)

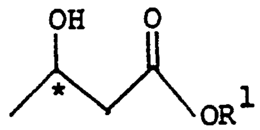

(I)

dans laquelle $R^1$ représente un groupe alkyle en $C_1$ à $C_4$, ou un dérivé de 3-[1-hydroxyéthyl]azétidine-2-one, de formule (II):

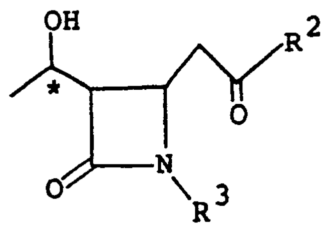

dans laquelle $R^2$ représente un groupe alkoxy en $C_1$ à $C_4$, un groupe arylalkoxy en $C_7$ à $C_8$ un groupe de formule (III):

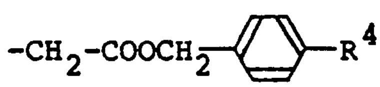

(III)

dans laquelle $R_4$ représente un atome d'hydrogène ou un groupe nitro, ou un groupe de formule (IV):

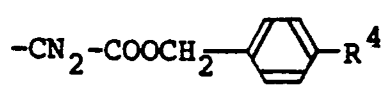

(IV)

dans laquelle $R^4$ est tel que défini ci-dessus; $R^3$ représente un atome d'hydrogène ou un groupe protecteur du groupe amino, et le composé fluoré obtenu, est un ester de l'acide 3-fluorobutyrique, de formule (V):

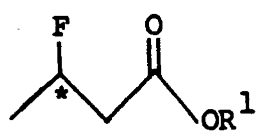

(V)

dans laquelle $R^1$ est tel que défini ci-dessus, et la configuration de l'atome de carbone fluoré, est inversée, ou un dérivé de 3-[1-fluoroéthyl]azétidine-2-one, de formule (VI):

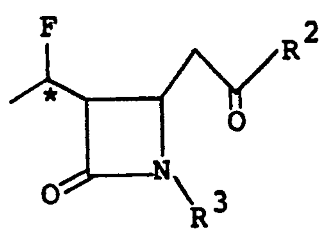

(VI)

dans laquelle $R^2$ et $R^3$, sont tels que définis ci-dessus, et la configuration de l'atome de carbone fluoré, est inversée.

3. Procédé selon la revendication 2, dans lequel le composé comportant le groupe hydroxyle, est un ester de l'acide 3(S)-hydroxy-butyrique, de formule (Ia):

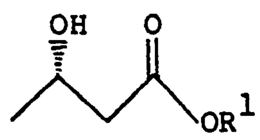

(Ia)

dans laquelle $R^1$ est tel que défini ci-dessus, et le composé fluoré obtenu, est un ester de l'acide 3(R)-fluoro-butyrique, de formule (Va):

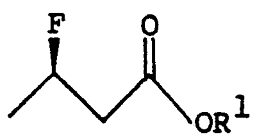

(Va)

dans laquelle $R^1$ est tel que défini ci-dessus.

4. Procédé selon la revendication 2, dans lequel le composé comportant le groupe hydroxyle, est un dérivé de 3-[1(S)-hydroxyéthyl]-azétidine-2-one, de formule (IIa):

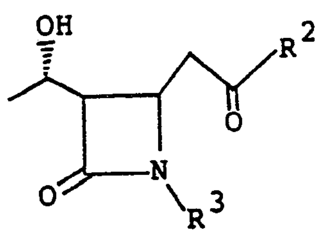

(IIa)

dans laquelle $R^2$ et $R^3$ sont tels que définis ci-dessus, et le composé fluoré obtenu, est un dérivé de 3-[1(R)-fluoroéthyl]-azétidine-2-one, de formule (VIa):

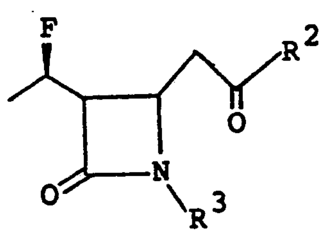

(VIa)

dans laquelle $R^2$ et $R^3$ sont tels que définis ci-dessus.